# EUROPEAN PATENT APPLICATION

(11) **EP 4 513 507 A1**
(43) Date of publication of application: **26.02.2025**
(21) Application number: 23193148.6
(22) Date of filing: 24.08.2023
(51) Int. Cl.: G16H 40/60, G16H 20/70, A61M 21/00

(54) **METHOD TO GENERATE PATIENT PERCEPTION CONTENT FOR GENTLE TRANSITION INTO/FROM ANESTHESIA**

(71) Applicant: Koninklijke Philips N.V., 5656 AG Eindhoven (NL)
(72) Inventor: VAN EE, Raymond, Eindhoven (NL); GEURTS, Lucas Jacobus Franciscus, Eindhoven (NL); KLAASSEN, Remy, 5656AG Eindhoven (NL); SPELT, Hanne Adriana Alijda, Eindhoven (NL); BULUT, Murtaza, Eindhoven (NL)
(74) Representative: Philips Intellectual Property & Standards

(57) **Abstract**

The present invention relates to a system and a method for creating a simulated environment for a subject undergoing and waking up from anesthesia. The system comprises a stimulation module (12), a subject monitoring module (14), and a controller (16). The stimulation module (12) comprises one or more sensory stimulation devices configured to apply at least one sensory stimulus over a subject to simulate an environment that will be experienced by the subject undergoing and waking up from anesthesia. The subject monitoring module (14) comprises one or more sensors configured to acquire sensor data from the subject. The controller (16) is configured to determine a sedation state of the subject based on the acquired sensor data and to control at least one of the one or more sensory simulation devices to modify the simulated environment based on the sedation state of the subject. The system may be used to create an ambient experience for a gentle transition into and/or from anesthesia. For example, the system may create an adaptive elements in the story to prevent or reduce the subject's mental confusion, thereby increasing temporal structure, so that the risk for getting delirium becomes smaller. In addition, the system may reduce anxiety upon the waking up. Furthermore, by reducing the subject's mental confusion and anxiety, the length of stay in the hospital may also be reduced.

## Description

### FIELD OF THE INVENTION

The present invention relates to a system and a method for creating a simulated environment for a subject undergoing and waking up from anesthesia.

### BACKGROUND OF THE INVENTION

Waking up from general anesthesia comes with the potential problem of experiencing delirium. Delirium is a mental state in which the subject is confused, disoriented, and not able to think or remember clearly. One major underlying reason is a dysfunction of the biological clock. We may all recognize a dysfunction of our own biological clock upon a major jetlag after a long transatlantic flight.

After waking from anesthesia this lack of mindfulness may be even more severe than upon a jet lag because the biological clock has been completely reset. Especially older people and those with heart disease (e.g., congestive heart disease), Parkinson's disease (PD), Alzheimer's disease (AD) are vulnerable. The duration of general anesthesia correlates with a phase shift of the biological clock. Individuals with postoperative cognitive dysfunction may benefit from an optimized approach to (re)adjust the brain's pacemaker. Integration of marked light exposure rhythmicity in the daily routine of human subjects has been shown to ameliorate a number of measures associated with major depression and cognitive decline.

For many children, undergoing anesthesia is scary ("you are stiff with fear and then suddenly you fall asleep"). Waking up from anesthesia can lead to additional problems. For example, some children wake up after surgery and begin crying and become combative. They are often extremely frightened, disoriented and refuse to be comforted, even after being reunited with their parents. Some even hallucinate and become so agitated that they have to be restrained. Those reactions, called emergence delirium, may result from a brain dysfunction that increases the release of hormones that prompt "fight-or-flight," a sympathetic nervous system reaction. The temporary combativeness after surgery is a complication affecting up to half of anesthetized children.

Currently, colored light has been presented on the ceiling above the bed of the subject (e.g., Philips' vital sky) to help the subject to experience a circadian rhythm by presenting blue light in the morning and orange light in the evening. Another approach provides a lighting system with a special time program that regulates the body clock by realizing multiple light-dark cycles during the day.

### SUMMARY OF THE INVENTION

There may be a need to reduce the risk for getting delirium.

The object of the present invention is solved by the subject-matter of the independent claims, wherein further embodiments are incorporated in the dependent claims. It should be noted that the following described aspects of the invention apply also for the system and the method for creating a simulated environment for a subject undergoing anesthesia.

According to a first aspect of the present invention, there is provided a system for creating a simulated environment for a subject undergoing and waking up from anesthesia. The system comprises a stimulation module, a subject monitoring module, and a controller. The stimulation module comprises one or more sensory stimulation devices configured to apply at least one sensory stimulus over a subject to simulate an environment that will be experienced by the subject undergoing and waking up from anesthesia. The subject monitoring module comprises one or more sensors configured to acquire sensor data from the subject. The controller is configured to determine a sedation state of the subject based on the acquired sensor data and to control at least one of the one or more sensory simulation devices to modify the simulated environment based on the sedation state of the subject.

Accordingly, the system as disclosed herein may be used to create a patient perception content for a gentle transition into and/or from anesthesia. For example, the system may present a story using an audio-video system, and create an adaptive elements in the story to prevent or reduce the subject's mental confusion. In this way, temporal structure may be increased so that the risk for getting delirium becomes smaller. In addition, the system may reduce anxiety upon the waking up. Furthermore, by reducing the subject's mental confusion and anxiety, the length of stay in the hospital may also be reduced.

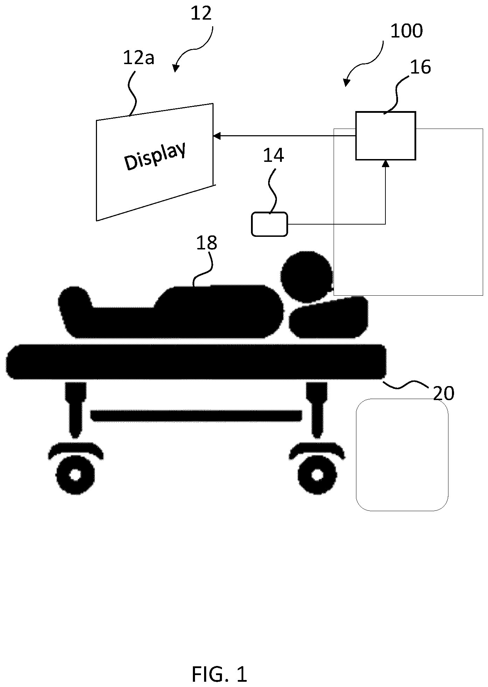

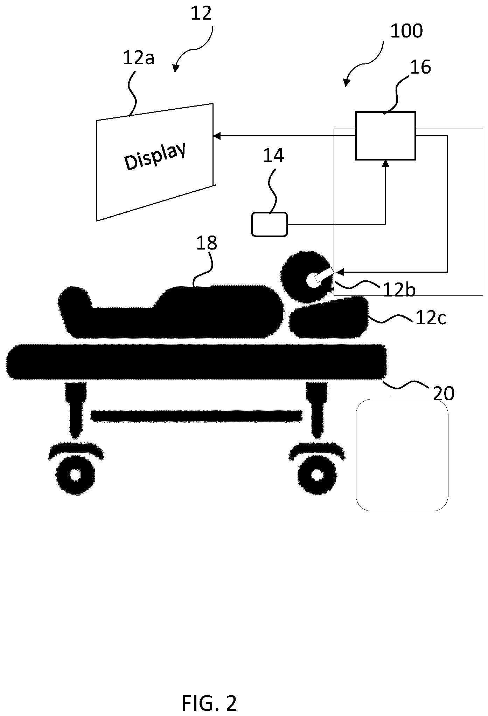

This will be described in detail hereinafter and in particular with respect to the exemplary systems shown in Figs. 1 and 2.
According to an embodiment of the present invention, the controller is configured to stop the simulated environment when it is determined from the acquired sensor data that the subject starts being unconscious, and to continue the simulated environment when it is determined from the acquired sensor data that the subject wakes up from anesthesia.

For example, if t0 is the time when the subject goes to sleep and t1 specifies the start of the waking up transition, the system may stop the ongoing story at t0 and then continue from where left at t1. To anticipate that the subject will go sleep at t0, it is possible to determine a transition period to slow down the story, and emphasize the elements at the continuation. T2 specifies when the subject will be fully awake. Between the time point t1 and the time point t2, where the subject is still trying to wake up, it is possible to slowly and gradually increase the simulation.

According to an embodiment of the present invention, the controller is configured to determine a likelihood that the subject will start being unconscious, during the anesthesia procedure, and/or wake up within a predetermine period of time, and to control at least one of the one or more sensory simulation devices to modify the simulated environment based on the determined likelihood. For example, the controller may receive the sensor data, e.g., motion data and/or physiological signals and may combine the received motion data and/or physiological signals in a probabilistic way, e.g. through Bayesian combination, to determine this likelihood of the onset moment that the subject will wake up.

According to an embodiment of the present invention, the one or more sensory stimulation devices comprises one or more of:
- a visual stimulation device configured to provide a visual content;
- an auditory stimulation device configured to provide an auditory content;
- a tactile stimulation device; and
- an olfactory stimulation device.

Examples of the visual stimulation device may include, but are not limited to, a display, a projector, and a head mounted display.

Examples of the auditory stimulation device may include, but are not limited to, a speaker and a headphone.

The stimulation module may comprise a tactile stimulation device, e.g., a pillow or matrass that slightly moves to simulate the environment that will be experienced by the subject.

The olfactory stimulation device may add olfaction to the stimulated environment with the use of aerosols and atomizers.

According to an embodiment of the present invention, the controller is configured to control the stimulation module to modify the simulated environment by:
- adapting a character's behaviour and/or a scenery element in the simulated environment based on the sedation state of the subject;
- speeding up or slowing down at least one sensory content provided by the one or more sensory stimulation devices based on the sedation state of the subject; and/or
- making the simulated environment more immersive or less immersive based on the sedation state of the subject.

According to an embodiment of the present invention, the stimulation module comprises a plurality of sensory stimulation devices. The controller is configured to generate a multisensory rhythmically synchronous signal to control the plurality of sensory stimulation devices to provide an immersive environment.

For example, it is possible to create more immersion, like e.g. adding tactile, sound, light and/or other multi sensorial effects to the simulated environment.

According to an embodiment of the present invention, the stimulation module is configured to simulate an environment using a psychological priming technique.

For example, prior to sleep it is possible to prime the subject with specific objects, colors, sounds, etc. that are associated with the presented story. Those elements will then be gradually brought back in congruence upon the waking up process.

According to an embodiment of the present invention, the stimulation module is configured to simulate an environment using a deep sleep stimulation method.

For example, deep sleep stimulation methods, such as periodic beep sounds (e.g., Philips' power sleep headset), may be used. In this way the amount of anesthesia chemicals can be reduced, and this can in turn reduce part of the side effects.

According to an embodiment of the present invention, the subject monitoring module comprises one or more of the following sensors:
- a motion sensor configured to acquire motion data of the subject; and
- a physiological sensor configured to acquire physiological data of the subject.

For example, the motion sensor may be a camera configured to detect a physical motion of a body part of the subject. The physiological sensor may acquire physiological signals from the subject including, but not limited to, eye movements, cortisol, heart rate, temperature, skin conductance, etc.

According to an embodiment of the present invention, the controller is configured to determine a phase shift in a subject's biological clock based on information on a circadian rhythm of the subject.

For example, the core body temperature of the subject may be measured during multiple days prior to anesthesia to determine the circadian rhythm of the subject prior to anesthesia. With the information about the phase shift in the subject's biological clock, the simulated environment may be adapted in order to regulate the subject's body block.

According to an embodiment of the present invention, the controller is configured to control the visual stimulation device to modify the simulated environment by:
- adapting one or more of a main color, brightness, and saturation in the visual content to the phase shift in the subject's biological clock; and/or
- integrating one or more food intake moments into the simulated environment.

For example, based upon the measured acute current phase shift in the subject's biological clock, the main colors in the visual content of the video may be shifted toward blue or towards orange.

For example, it is possible to include breakfast into the story. In the story the character could for example start making breakfast.

According to an embodiment of the present invention, the controller is configured to determine a moment to scan the subject during a natural sleep based on the circadian rhythm of the subject, and to determine a moment to administer anesthesia.

For example, the circadian rhythm can be used to determine the appropriate moment to scan the subject during sleep (e.g., deep sleep state) and the sensory data can be used to determine if a subject will stay in the sleep phase long enough. In addition, when the rhythm is determined, a subject close to the correct sleep stage could be brought to sleep while being in the scanner or in the scanner room using the ambient lighting, sound and video. Furthermore, if a subject's sleep rhythm is known and used correctly, a minimum of sedatives could be enough to bring the subject to a resting state instead of having to use full anesthesia.

According to an embodiment of the present invention, the stimulation module is configured to provide the simulated environment that is specific for a body part of the subject, and/or based on a subject profile and/or a subject preference.

For example, the character may have an injury that resembles the injury of the subject (e.g., a knee injury for a knee surgery, or a head injury for a head scan).

For example, the story may be personalized to specific use-cases, e.g., Disney for kids, reassuring messages for adults, etc. This may include adding objects and people that the subject is familiar with.

According to an embodiment of the present invention, the auditory simulation device is configured to simulate an environment with a soundscape that resembles a soundscape of a surgery environment and/or a scan environment.

For example, many subjects require general anesthesia or sedation for MRI because of the need to minimize movement during potentially long scanning times and the noisy and claustrophobic environment of the scanner. The start of anesthesia may be reflected in the behavior of the character in the simulated environment. For example, the character will find a place to sleep in an environment with a soundscape that resembles the soundscape of the surgery environment and/or scan environment, thus creating a seamless adaptive continuation of the story within the simulated environment.

According to a second aspect of the present invention, there is provided a method for creating a simulated environment for a subject undergoing and waking up from anesthesia, the system comprising:
- applying at least one sensory stimulus over a subject to simulate an environment that will be experienced by the subject undergoing and waking up from anesthesia;
- acquiring sensor data from the subject in the simulated environment; and
- determining a sedation state of the subject based on the acquired sensor data and to control at least one of the one or more sensory simulation devices to modify the simulated environment based on the sedation state of the subject.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

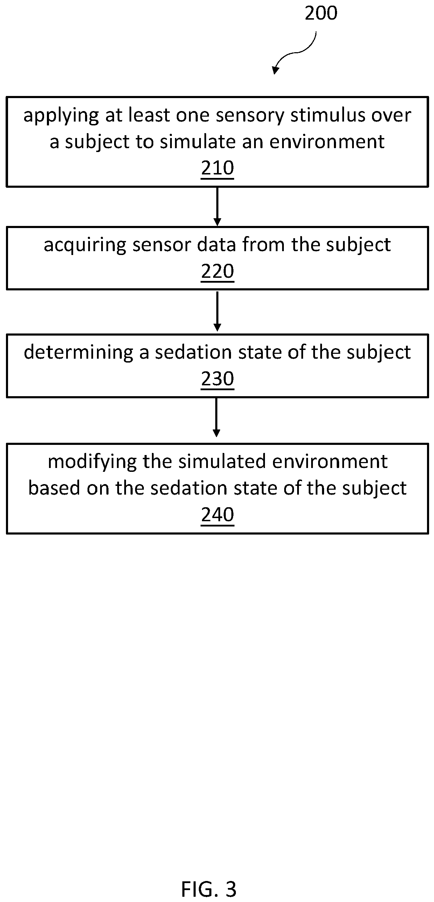

This will be described in detail hereinafter and in particular with respect to the exemplary system shown in Fig. 3.

According to another aspect, there is provided a computer program product comprising instructions to cause the system according to the first aspect to execute the steps of the method according to the second aspect and any associated example.

According to a further aspect, there is provided a computer-readable medium having stored thereon the computer program.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 shows schematically and exemplarily an example of a system for creating a simulated environment for a subject undergoing anesthesia.
Fig. 2 shows schematically and exemplarily a further example of a system for creating a simulated environment for a subject undergoing anesthesia.
Fig. 3 illustrates a flowchart of an exemplary method.

### DETAILED DESCRIPTION OF EMBODIMENTS

Fig. 1 shows schematically and exemplarily an example of a system 100 for creating a simulated environment for a subject 18, e.g., a patient, undergoing and waking up from anesthesia. In an example, the term "anesthesia" may include sedation and general anesthesia. General anesthesia is a state of complete loss of consciousness. Sedation is a state somewhere between very sleeping in consciousness and yet not unconscious.

The system comprises a stimulation module 12, a subject monitoring module 14, and a controller 16, which may communicate with each other via a wired and/or wireless connection. As shown in Fig. 1, the subject 18 may be supported by a mobile subject support 20, which may be a bed shown in Fig. 1, a wheelchair, or an autonomous movement unit, for transferring the subject 18 from one location to another within a healthcare facility. The stimulation module 12 and the subject monitoring module 14 may be attached, e.g., removably attached, to the mobile subject support 20.

The stimulation module 12 comprises one or more sensory stimulation devices configured to apply at least one sensory stimulus over a subject to simulate an environment that will be experienced by the subject undergoing anesthesia. In some examples, the stimulation module 12 may comprise a visual stimulation device. The visual stimulation device may be a display shown in Fig. 1, such as a head mounted display, for providing a visual content. The visual content may include any content format that features or includes video, such vlogs, animated GIFs, live videos, films, television programs or any combination thereof. In some examples (not shown), the visual stimulation device may be a projector configured to project the visual content projects onto a screen or wall. In some examples, the stimulation module may comprise an auditory stimulation device, such as a speaker or a headphone configured to provide an auditory content. In some examples (not shown), the stimulation module 12 may comprise a tactile stimulation device, e.g., a pillow or matrass that slightly moves to simulate the environment that will be experienced by the subject. In some examples (not shown), the stimulation module 12 may comprise an olfactory stimulation device, which may add olfaction to the stimulated environment with the use of aerosols and atomizers.

In some further examples, the stimulation module 12 may comprise a plurality of sensory stimulation devices 12 and the controller 16 is configured to generate a multisensory rhythmically synchronous signal to control the plurality of different sensory stimulation devices to provide an immersive environment. For example, Fig. 2 shows schematically and exemplarily a further example of a system 100 for creating a simulated environment for a subject 18 undergoing anesthesia. In the example illustrated in Fig. 2, in addition to the visual stimulation device 12a, the stimulation module 12 further comprises an auditory stimulation device, such as the headphone shown in Fig. 2, configured to provide an auditory content, and a tactile stimulation device 12c, such as the pillow shown in Fig. 2, which may slightly move in the story of the character. In the example illustrated in Fig. 2, the controller 16 may generate multisensory rhythmically synchronous signals (e.g., tactile, sound, vision) in order to provide an immersive environment. For example, it is possible to combine visible sea, breaking wave sound, and the movement of the pillow to create multi sensorial effects.

Turning back to Fig. 1, the subject monitoring module 14 comprises one or more sensors configured to acquire sensor data from the subject. In some examples, the subject monitoring module 14 may comprise one or more motion sensors configured to acquire motion data of the subject for sedation depth measurement. For example, a camera may be used as the motion sensor to detect the physical motion of one or more body parts of the subject. In some examples, the subject monitoring module 14 may comprise one or more physiological sensors configured to acquire physiological data of the subject to measure a level of sedation. Examples of the physiological signals from the subject may include, but are not limited to, eye movements, cortisol, heart rate, temperature, skin conductance, etc. In some further examples, the subject monitoring module 14 may comprise both motion and physiological sensors.

In some implementations, a remote haptic device may be used to mimic a very tactile nudge or a touch and/or a wearable skin trigger with heating and/or cooling to induce a pain sensation. The remote haptic device may be modulated to generate a specific physical form of nudge/tap at specific locations, i.e. impact regions, at cheeks, forehead, hand, etc. The impact region to generate the localized stimulus may be determined based on the assessment description of sedation as defined in various sedation determining scales. A range of remote haptic stimuli may be used, including, but not limited to, acoustic fields (e.g. ultrasound), electromagnetic fields (e.g. light), aerodynamic fields (e.g. air flow), magnetic fields, etc. Since they are bidirectional, they can also be used for closed loop monitoring to, assess the impact of nudging, its response and compare with expected sedation level awareness. Subject reactions in response to the haptic sensations may include, but are not limited to, a movement of a body part with the impact region, a change in a facial gesture of the subject, a change in a measured vital sign, a muscle response associated with the body part with the impact region, and a nerve response associated with the body part with the impact region. The movement of the body part and the change in the facial gesture may be detected with a camera or video based system. The vital sign may be monitored by a galvanic skin response (GSR) sensor. The muscle/nerve response may be detected by an electromyography (EMG) or electroencephalography (EEG) sensor. The sedation level may be determined by correlating the detected subject reaction for a haptic feedback with an anticipated response.

In some implementations, skin triggers with heating and/or cooling may be used. The measurement may be done with the help of an actuator device that triggers subject feedback in a well-defined way. The actuator could be a heating/cooling device, which at an exact defined position generates a defined input signal to the subject, thereby leading to a reaction of the subject that is correlated to the subject's sedation level.

The controller 16 may be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).These and other aspects of the present invention will become apparent from and be elucidated with reference to the embodiments described hereinafter. In various implementations, the controller 16 may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

The controller 16 is configured to determine a sedation state of the subject based on the acquired sensor data and to control at least one of the one or more sensory simulation devices to modify the simulated environment based on the sedation state of the subject. As an example, the simulated environment may be adapted upon the measured sedation state of the subject in a way that helps to limit the risks of delirium or emergency delirium. In some examples, a character's behavior and/or a scenery element may be adapted to solicit a physiological or a mental effect. For example, once the start of anesthesia is determined from motion data and/or physiological signals acquired from the subject, this start of anesthesia may be then reflected in the behavior of the character in the simulated environment. One possibility, among many others, is that the character finds a place to sleep, once the start of anesthesia is determined. Alternatively or additionally, it is possible to speed up or slow down the simulated environment based on the sedation state of the subject. For example, the story could go slower, giving more ease to adapt to the new reality. Also other scenery elements can adapt and create more immersion, like e.g. adding tactile, sound, light and other multi sensorial effects.

The system 100 may be used to create an ambient experience for a gentle transition into and/or from anesthesia. For example, the system 100 may create an adaptive elements in the story to prevent or reduce the subject's mental confusion, thereby increasing temporal structure. In this way, the risk for getting delirium becomes smaller. In addition, the system 100 may reduce anxiety upon the waking up. Furthermore, by reducing the subject's mental confusion and anxiety, the length of stay in the hospital may also be reduced.

Anesthesia may be required in many scan procedures and interventional procedures, particularly for longer scans or particular subject groups, e.g., claustrophobic subjects. It should be noted that the system and the method as disclosed herein are general and not specific for specific scan procedures and/or devices. For example, the system and the method as disclosed herein may be used for e.g., Magnetic Resonance Imaging (MRI), Image Guided Therapy (IGT), X-ray imaging, Computed Tomography (CT), UltraSound, etc. It is possible that either a scan procedure or a device requires a specific anesthesia procedure with possibly multiple anesthesia phases, entailing a story that contains multiple sleep phases. Stories may also be specific for the body part that is to be scanned, such as a head injury for a head scan. In addition, stories can be personalized depending on the subject profile and subject preferences. In some examples, the stories may be personalized to specific use-cases e.g., Disney stories for kids, reassuring messages for adults, etc. In some examples, the stories may be personalized to a specific subject to avoid personal anxiety situations (e.g., a specific subject may be afraid of crossing a bridge). In some examples, the adaptive stories may be influenced by the subject, e.g., through the measurements of sleep-related and consciousness-related signals and/or through a choice panel, with which the subject may e.g., consciously chose to go back to sleep.

In some examples, the controller may be configured to stop the simulated environment when it is determined from the acquired sensor data that the subject starts being unconscious, and to continue the simulated environment when it is determined from the acquired sensor data that the subject wakes up from anesthesia. For example, if t0 is the time when the subject goes to sleep and t1 specifies the start of the waking up transition, the system 100 may stop the ongoing story at t0 and then continue from where left at t1. To predict that the subject will go sleep at t0, it is possible to determine a transition period to slow down the story, and emphasize the elements at the continuation. t2 specifies when the subject will be fully awake. Between the time point t1 and the time point t2, where the subject is still trying to wake up, it is possible to slowly and gradually increase the simulation. The time points t0, t1, and t2 may be estimated based on the sedation state of the subject. For example, the controller 16 may be configured to determine a likelihood that the subject will start being unconscious, during the anesthesia procedure, and/or wake up within a predetermine period of time, e.g., through Bayesian combination, and to control at least one of the one or more sensory simulation devices to modify the simulated environment based on the determined likelihood.

In the following, an exemplary operation of the system 100 will be described with respect to an MRI scan procedure. However, it will be appreciated that the system 100 can also be used in any clinical procedure, including various surgery environments and/or scan environments, such as IGT, X-ray, CT, UltraSound, etc.

### Prior to anesthesia

Prior to an MRI scan procedure, anesthesia may be induced in a dedicated anesthetic room adjacent to the MRI scanner but outside the 5G contour. This provides an area in which standard anesthetic and resuscitation equipment can be stored and used.

The stimulation module 12 may comprise one or more sensory stimulation devices to provide a half or full immersive audio-visual system for providing an interactive audio-visual environment to the subject 18 undergoing anesthesia. For example, the stimulation module may comprise a display shown in Fig. 1 to present a story using a visual content. This may possibly be about a traveling character. In addition to the story, the interactive audio-visual environment may provide the required support for the sedated subject, such as audio information and/or video guidance, to the subject to reduce the confusion of the subject and thus the risk of anxiety of the subject.

The subject monitoring module 14 comprises one or more sensors configured to acquire sensor data, e.g., motion data and/or physiological signals, from the subject 18.

The controller 16 receives the sensor data, e.g., motion data and/or physiological signals, and may combine the received motion data and/or physiological signals in a probabilistic way, e.g. through Bayesian combination, to determine this likelihood of the offset moment that the subject will start being unconscious. Based on the sedation state of the subject 18, the controller 16 may control at least one of the one or more sensory simulation devices, e.g., the display 12a shown in Fig. 1 or the display 12a, the headphone 12b, and the pillow 12c shown in Fig. 2, to modify the simulated environment based on the sedation state of the subject. For example, along the journey of this character, anesthesia starts having effect. Once start of anesthesia is determined from the sensor data, e.g., motion data and/or physiological signals, this start of anesthesia may be then reflected in the behavior of the character. One possibility, among many others, is that the character finds a place to sleep. In a related embodiment the character will find a place to sleep in an environment with a soundscape that resembles the soundscape of the scan environment (e.g., MRI scan environment) and/or the surgery environment. Stories may also be specific for the body part that is to be scanned and/or to be operated. For example, the character will have an injury that resembles the injury of the subject, e.g. a knee injury for a knee surgery, or a head injury for a head scan.

### During anesthesia

The subject 18 will be transferred into the scanning room on the mobile subject support 20 during anesthesia. During the MRI scan procedure, maintenance of anesthesia can be achieved through e.g., inhalation techniques.

During anesthesia, the stimulation module 12 may further stimulate the subject 18 when the subject 18 is unconscious. Also, in case the subject 18 starts waking up too early, the controller 16 may be configured to make the Ambient Experience more immersive e.g., adding tactile, sound, light and other multisensorial effects to create more immersion, in turn reducing anesthesia-awareness trauma.

### Upon waking up after anesthesia

After the MR scan procedure, the subject 18 will be transferred to a wake-up room. The controller 16 receives the sensor data, e.g., motion data and/or physiological signals and may combine the received motion data and/or physiological signals in a probabilistic way, e.g. through Bayesian combination, to determine this likelihood of the onset moment that the subject will wake up. Based on the sedation state of the subject 18, the controller 16 may control at least one of the one or more sensory simulation devices, e.g., the display 12a shown in Fig. 1 or the display 12a, the headphone 12b, and the pillow 12c shown in Fig. 2, to modify the simulated environment based on the sedation state of the subject. For example, upon this onset moment of the video, the story may continue with the sleeping character. When the subject 18 indeed wakes up, the story character may also wake up at the same location where the subject 18 went to sleep. Depending on the state of the subject 18, i.e. is there indeed a delirium setting on, or is the subject quickly conscious, the story may unfold in a slower and/or quicker way. This may also involve less story elements and/or less sensory stimulation. The story (e.g., Disney story) and theme could come back here in light and soundscapes (e.g., nemo soundscape). The positive emotional response (e.g., dopamine) associated with the story may help to alleviate the delirium problems mentioned above. This state may be partly determined by the above sleep-related signals, but may also be determined by a nurse via a camera.

In some implementations, the subject monitoring module 14 may continuously monitor e.g., motion and/or physiological signals from the subject, and upon the detection of a predetermined pattern of variation, the controller 16 may modify control the stimulation module 12 to modify the simulated environment, e.g., making the story less exciting. In some examples, the system 100 may enter a biofeedback loop, where the sensor data, such as motion and/or physiological signals (e.g., breathing or heart rate) may be used as input to control a story element over a longer period of time, e.g., a music track or a sky that moves, in order to help to calm down or control the rhythm of breathing or heart rate in a predefined way. At some point based upon waking- and delirium-related signals, adaptive elements become less and less and when possible we bring the subject back to reality.

In some implementations, it is possible to use psychological priming techniques, where prior to sleep it may prime the subject with specific objects, colors, sounds, etc. that are associated with the presented story. Those elements may then be gradually brought back in congruence upon the waking up process.

In some implementations, it is also possible to use deep sleep stimulation methods e.g., periodic beep sounds (e.g., Philips' power sleep headset). In this way, the amount of anesthesia chemicals can be minimized, and this can in turn minimize part of the side effects.

In some cases, multiple scan procedures may be required. In these cases, the simulated environment (e.g., with a story) may continue so that sequential visits for the scans (e.g., MR scans of MR therapy), with associated anesthesia, become part of the story. Preferably, the need for anesthesia will become less and less over time because the subject becomes less and less anxious for a scan within the bore of a MR scanner. Thus, depending on the severity of anesthesia, at each visit the simulated environment can real-time be adapted.

In some implementations, the controller 16 may additionally control at least one of the one or more sensory simulation devices to modify the simulated environment to help the subject to regulate the body block. In these implementations, the subject monitoring module 14 may comprise at least one sensor configured to acquire data indicative of a circadian rhythm of the subject, and the controller 16 is configured to determine a phase shift in a subject's biological clock based on the acquired data. For example, the core body temperature of the subject may be measured during multiple days prior to anesthesia to determine the circadian rhythm of the subject prior to anesthesia. For example, the subject may be an early bird (i.e., morning active person) or a night owl (i.e., evening active person), and/or a subject could have a shifted circadian rhythm (e.g., possibly to a recent transatlantic flight) and or biphasic circadian rhythm. The sensor may also be used to measure the core body temperature of the subject e.g., after the surgery to determine the remaining acute circadian rhythm and its phase shift.

Based on the determined phase shift in a subject's biological clock, the controller 16 may control the visual stimulation device to modify the simulated environment by adapting one or more of a main color, brightness, and saturation in the visual content to the phase shift in the subject's biological clock. For example, based upon the measured acute current phase shift in the subject's biological clock, the main colors in the visual content of the video may be shifted toward blue or towards orange. If the phase shift of the subject's biological clock indicates that the clock is in morning-mode the wavelengths of colors used can be shifted towards blue. On the other hand, if the phase shift of the subject's biological clock indicates that the clock is in evening-mode the wavelengths of colors used can be shifted towards orange. Alternatively, it is possible to use colors that fit the solar light-dark cycle, possibly taking into account whether the subject appeared to be a morning or evening person from the data collected prior to anesthesia. The influencing of a 24h daily structure may be refined by applying additional elements to the visual content. Alternatively or additionally, based on the determined phase shift in a subject's biological clock, the controller 16 may control the visual stimulation device to modify the simulated environment by integrating one or more food intake moments into the simulated environment. For example, the visual content may include food intake moments to integrate specific moments with a 24 rhythm.

Scanning pediatric subjects may be challenging and often anesthesia is applied to make sure the subjects lie still during the entirety of the scan. However, alternatively subjects are also being scanned during natural sleep, omitting the drawbacks of anesthesia (e.g., health risks, logistics). The circadian rhythm can be used to determine the best moment to scan the subject during sleep (deep sleep state) and the sensor data can be used to determine if a subject will stay in the sleep phase long enough. In addition, when the rhythm is determined, a subject close to the correct sleep stage could be brought to sleep while being in the scanner or in the scanner room using the ambient lighting, sound and video. Furthermore, if a subject's sleep rhythm is known and used correctly, a minimum of sedatives could be enough to bring the subject to a resting state instead of having to use full anesthesia.

Fig. 3 illustrates a flowchart describing an exemplary method 200 according to an embodiment of the present disclosure.

At block 210, the method 200 comprises the step of applying at least one sensory stimulus over a subject to simulate an environment that will be experienced by the subject undergoing and waking up from anesthesia.

In some examples, a single sensory stimulus, e.g., visual stimulus, may be provided.

In some examples, two or more sensory stimuli are applied over the subject. A multisensory rhythmically synchronous signal may be generate to synchronize the two or more sensory stimuli to provide an immersive environment.

At block 220, the method further comprises the step of acquiring sensor data from the subject in the simulated environment. The sensor data may comprise motion data of the subject and/or physiological data of the subject.

At block 230, the method comprises the step of determining a sedation state of the subject based on the acquired sensor data.

At block 240, the method 200 further comprises the step of controlling at least one of the one or more sensory simulation devices to modify the simulated environment based on the sedation state of the subject. For example, the personalized story may be adapted upon the measured signals of the subject in a way that helps to limit the risks of (emergency) delirium. In this story e.g. a character's behavior or scenery elements are adapted to solicit a physiological or a mental effect. Alternatively, the story could go slower, giving more ease to adapt to the new reality. Also other elements may adapt and create more immersion, like e.g. adding tactile, sound, light and other multi sensorial effects.

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, and/or method described herein. In addition, any combination of two or more such features, systems, and/or methods, if such features, systems, and/or methods are not mutually inconsistent, is included within the inventive scope of the present disclosure.

## Claims

1. A system for creating a simulated environment for a subject undergoing and waking up from anesthesia, the system comprising:
- a stimulation module (12) comprising one or more sensory stimulation devices configured to apply at least one sensory stimulus over a subject to simulate an environment that will be experienced by the subject undergoing and waking up from anesthesia;
- a subject monitoring module (14) comprising one or more sensors configured to acquire sensor data from the subject; and
- a controller (16) configured to determine a sedation state of the subject based on the acquired sensor data and to control at least one of the one or more sensory simulation devices to modify the simulated environment based on the sedation state of the subject.

2. The system according to claim 1,
wherein the controller is configured to stop the simulated environment when it is determined from the acquired sensor data that the subject starts being unconscious, and to continue the simulated environment when it is determined from the acquired sensor data that the subject wakes up from anesthesia.

3. The system according to claim 1 or 2,
wherein the controller is configured to determine a likelihood that the subject will start being unconscious, during the anesthesia procedure, and/or wake up within a predetermine period of time, and to control at least one of the one or more sensory simulation devices to modify the simulated environment based on the determined likelihood.

4. The system according to any one of the preceding claims,
wherein the one or more sensory stimulation devices comprise one or more of:
- a visual stimulation device (12a) configured to provide a visual content;
- an auditory stimulation device (12b) configured to provide an auditory content;
- a tactile stimulation device (12c); and
- an olfactory stimulation device.

5. The system according to any one of the preceding claims,
wherein the controller is configured to control the stimulation module to modify the simulated environment by:
- adapting a character's behaviour and/or a scenery element in the simulated environment based on the sedation state of the subject;
- speeding up or slowing down at least one sensory content provided by the one or more sensory stimulation devices based on the sedation state of the subject; and/or
- making the simulated environment more immersive or less immersive based on the sedation state of the subject.

6. The system according to any one of the preceding claims,
wherein the stimulation module comprises a plurality of sensory stimulation devices; and wherein the controller is configured to generate a multisensory rhythmically synchronous signal to control the plurality of sensory stimulation devices to provide an immersive environment.

7. The system according to any one of the preceding claims,
wherein the stimulation module is configured to simulate an environment using a psychological priming technique.

8. The system according to any one of the preceding claims,
wherein the stimulation module is configured to simulate an environment using a deep sleep stimulation method.

9. The system according to any one of the preceding claims,
wherein the subject monitoring module comprises one or more of the following sensors:
- a motion sensor configured to acquire motion data of the subject; and
- a physiological sensor configured to acquire physiological data of the subject.

10. The system according to any one of the preceding claims,
wherein the controller is configured to determine a phase shift in a subject's biological clock based on infomration on a circadian rhythm of the subject.

11. The system according to claim 10,
wherein the controller is configured to control a visual stimulation device to modify the simulated environment by:
- adapting one or more of a main color, brightness, and saturation in a visual content to the phase shift in the subject's biological clock; and/or
- integrating one or more food intake moments into the simulated environment.

12. The system according to claim 10 and 11,
wherein the controller is configured to determine a moment to scan the subject during a natural sleep based on the circadian rhythm of the subject, and to determine a moment to administer anesthesia.

13. The system according to any one of the preceding claims,
wherein the stimulation module is configured to provide the simulated environment that is specific for a body part of the subject, and/or based on a subject profile and/or a subject preference.

14. The system according to any one of the preceding claims,
wherein the stimulation module comprises an auditory simulation device configured to simulate an environment with a soundscape that resembles a soundscape of a surgery environment and/or a scan environment.

15. A method (200) for creating a simulated environment for a subject undergoing and waking up from anesthesia, the system comprising:
- applying (210) at least one sensory stimulus over a subject to simulate an environment that will be experienced by the subject undergoing and waking up from anesthesia;
- acquiring (220) sensor data from the subject in the simulated environment;
- determining (230) a sedation state of the subject based on the acquired sensor data; and
- controlling (240) at least one of the one or more sensory simulation devices to modify the simulated environment based on the sedation state of the subject.
